Europäisches Patentamt

European Patent Office

Office européen des brevets

(11) Publication number: **0 402 132 B1**

## EUROPEAN PATENT SPECIFICATION

(45) Date of publication of patent specification: **21.12.94**　(51) Int. Cl.⁵: **C12Q 1/70**, C12Q 1/68, //C12N15:37

(21) Application number: **90306185.1**

(22) Date of filing: **07.06.90**

---

(54) Method for the detection of human papilloma-virus.

---

(30) Priority: **08.06.89 JP 144230/89**

(43) Date of publication of application:
**12.12.90 Bulletin 90/50**

(45) Publication of the grant of the patent:
**21.12.94 Bulletin 94/51**

(84) Designated Contracting States:
**DE FR GB**

(56) References cited:
**EP-A- 0 301 968**
**WO-A-88/06634**
**WO-A-89/09940**
**WO-A-90/02821**

**CANCER RESEARCH, vol. 48, 01 June 1988;
A. SCHNEIDER-GÄDICKE et al., pp.
2969-2974&NUM;**

**JOURNAL OF EXPERIMENTAL MEDICINE, vol.
167, January 1988, The Rockefeller University
Press; D.K. SHIBATA et al., pp. 225-230&NUM;**

(73) Proprietor: **TAKARA SHUZO CO. LTD.
609 Takenakacho
Fushimi-ku Kyoto (JP)**

(72) Inventor: **Shimada, Masamitsu
3-2-62-203, Ichiriyama
Otsu-shi, Shiga-ken (JP)**
Inventor: **Kato, Ikunoshin
1-1-150 Nanryo-cho
Uji-shi, Kyoto-fu (JP)**
Inventor: **Fukushima, Michio
10-4-11, Maruyama Nishi-machi,Chuo-ku
Sapporo-shi, Hokkaido (JP)**
Inventor: **Fujinaga, Kei
5-6-25 Asahigaoka,
Chuo-ku
Sapporo-shi, Hokkaido (JP)**

(74) Representative: **Marlow, Nicholas Simon et al
Reddie & Grose
16, Theobalds Road
London WC1X 8PL (GB)**

---

## Description

This invention relates to a method for the detection of specific DNA regions of human papilloma-virus (hereinafter referred to as HPV). This invention also relates to a detection kit which is to be used for this method.

One factor in the occurrence of cervical carcinoma has been considered to be HPV (M. Durst et al., Proc. Natl. Acad. Sci. USA, 80, 3812-3815, 1983). More than 50 types of HPV have been identified, and HPV 16 has been detected in 40-60% of the reported cases of this disease, HPV 18 in 10-20%, and other types of HPV, most often HPV 33, at a lower percentage.

A culture system for such viruses has not yet been established for use as a detection method for HPV, and immunological detection is difficult, because the production of virus particles cannot be detected when the virus is in the form of viral DNA (i.e., not packed into particles). For these and other reasons, conventional methods for detection mainly involve detection of the viral gene itself. With the Southern hybridization method, M. Dürst et al. found that some 60% of samples of tissue from cervical cancers contain HPV 16 or HPV 18 DNA. The sensitivity of the detection by Southern hybridization is at the level of 0.5-1.0 copy per cell. It has also been found that the DNA of HPV in tissues from cervical cancer is integrated into the human genome. In addition, the integrated DNA of HPV has, as a rule, a large number of deletions, and many cases have been found in which the only regions that are completely conserved of the original are the genes E6 and E7 (Schwarz et al., Nature, 314, 111-114, 1985).

With Southern hybridization, the method currently used for the detection of the DNA of HPV, detection is not possible unless there is at least 1 pg of DNA of HPV. This method for detection is also limited by the need for at least 100 mg or so of tissue sample.

In the precancerous stage, that of cervical intraepithelial neoplasia (CIN), there is progress through the stages CINI to CINII and then to CINIII before the stage of carcinoma is reached. In biopsies taken of precancerous tissues, only a very small amount of sample can be obtained, and by the currently used methods for detection, detection from these samples is often difficult.

Recently, the polymerase chain reaction (PCR) has been developed as a highly sensitive method for genetic diagnosis (Methods in Enzymology, 155, 335-350, 1987).

The PCR method can be used to amplify enzymatically and in a specific way only the target gene so that it is useful for the detection of the target gene, giving high specificity with only a small amount of sample.

In the PCR method, the enzyme used is, for example, heat-resistant enzyme Taq polymerase, and amplification is accomplished by a cycle of the step of denaturation of DNA at 94°C, followed by the step of annealing of primer DNA at 55°C and the step of enzymatic synthesis of complementary DNA chains at 72°C, which cycle is repeated the desired number of times at these temperatures, resulting in the exponential amplification of the target gene.

For example, by 25 times repetition of the temperature cycle, the target DNA is amplified about 100,000 times. This PCR method is the most useful for the detection of DNA of HPV with high sensitivity from a trace amount of sample.

The detection of HPV DNA with the use of PCR is known in the reports of Melchers et al. (Journal of Medical Virology, 27, 329-335, 1989), Tidy et al. (Lancet, 1, 434, 1989), Shibata et al. (Laboratory Investigation, 59, 555-559, 1988, Young et al. (British Medical Journal, 298, 14-18, 1989), Hertz et al. (Japanese Laid-Open Patent Application No. 128800/89) and Morris et al. (WO88/06634).

In the detection of HPV DNA in tissue from cervical cancer, which DNA has been integrated into the human genome, detection of the DNA regions E6 and E7, which are conserved entirely in the human genome, is most suitable. The greatest sensitivity possible with the PCR method is found by theoretical calculation to be the detection of $10^{-6}$ pg of DNA of HVP, but to achieve this maximum sensitivity; it is a prerequisite that the most suitable region of the viral DNA be chosen for amplification.

Also, so that each type of HPV can be identified type specifically, it is necessary to select a pair of primers for amplification which are type-specific, and to select probes for use in detection which are type-specific.

In addition, for the amplification and detection of the selected region or regions, the pair of primers selected and the selected probes must be suitable for the accurate detection of the presence of HPV in clinical specimens.

In the report of Melchers et al. mentioned above, the regions selected were L1 and E1-E2. As described above, the regions of HPV DNA other than E6 and E7 may have deletions when they are integrated into the human genome, and therefore, when regions L1 and E1-E2 are selected as the regions to be amplified during the detection of HPV, there is a risk of a negative result in cases in which HPV is actually present.

EP 0 402 132 B1

In the papers published by the groups of Tidy and of Shibata and of Young, the regions selected for amplification were region E6 and/or region E7, but the detection of HPV in normal tissue specimens of cervix was high, 30-84%. The frequency of the occurrence of type 16 and of type 18 HPV in normal cervical tissue is about 10% (Lancet 1, 703-705, 1987). The methods for the detection of HPV of the three groups mentioned above will probably give rise to problems when it is necessary to distinguish between healthy and diseased tissue in the clinical use of these methods.

The above cited patent applications of the groups of Hertz and of Morris do not mention the examination of clinical specimens.

The object of this invention is to provide a method for the detection of HPV DNA and to provide a kit which makes use of this method with which during the detection of the HPV DNA, the type of the virus is identified clearly, and the amplified region of HPV DNA is amplified with high sensitivity; also this method offers a standard for decisions about the malignancy of cervical tissue in terms of whether there is cervical cancer, a precancerous condition, or neither.

Briefly, this invention firstly relates to a method for the detection of HPV 16, HPV 18, HPV 33, or some combination of these viruses, which method makes use of amplification with the use of a pair of oligonucleotide primers consisting of at least one DNA region selected from part or all of the E6 or E7 regions of the HPV 16, HPV 18, and HPV 33 genomes.

Secondly, this invention relates to a detection kit for the detection by the use of the above mentioned method, which kit is characterized by containing a probe for the detection of amplified DNA, and also the primer pair for amplification of a specific DNA region or regions.

According to the invention there is provided a method for the detection of human papilloma-virus HPV16, HPV 18, HPV 33 or any combination of these viruses by amplification of at least one HPV DNA region by use of a pair of oligonucleotide primers characterised in that the DNA region has a structure shown in Formulae 1 to 6:

Region I

5'- AAGGGCGTAACCGAAATCGGTTGAAC
CGAAACCGGTTAGTATAAAAGCAGAC
ATTTTATGCACCAAAAGAGAACTGCA
ATGTTTCAGGACCCACAGGAGCGACCC         HPV16
AGAAAGTTACCACAGTTATGCACAGA
GCTGCAAAC-3'                    [ 1 ]

5'- AAGGGAGTAACCGAAAACGGTCGGGA

CCGAAAACGGTGTATATAAAAGATGT
GAGAAACACACCACAATACTATGGCG         HPV18
CGCTTTGAGGATCCAACACGGCGACC
CTACAAGCTACCTGATCTGTGCACGG
AACTGAACAC-3'                   [ 2 ]

5'- TAGGGTGTAACCGAAAGCGGTTCAAC
CGAAAACGGTGCATATATAAAGCAAA
CATTTTGCAGTAAGGTACTGCACGAC         HPV33
TATGTTTCAAGACACTGAGGAAAAAC
CACGAACATTGCATGATTTGTGCCAAG
CATTGGAGAC-3'                   [ 3 ]

Region II

5'- GAGGAGGATGAAATAGATGGTCCAGCT
GGACAAGCAGAACCGGACAGAGCCCAT         HPV16
TACAATATTGTAACCTTTTGTTGCAAG
TGTGACTC-3'                     [ 4 ]

EP 0 402 132 B1

```
5'- GAAAACGATGAAATAGATGGAGTTAAT
    CATCAACATTTACCAGCCCGACGAGCC   HPV18
    GAACCACAACGTCACACAATGTTGTG
    TATGTGTTGTAAGTGTGAAGC- 3'   ( 5 )

5'- GAGGATGAAGGCTTGGACCGGCCAGA
    TGGACAAGCACAACCAGCCACAGCTG   HPV33
    ATTACTACATTGTAACCTGTTGTCACA
    CTTGTAACAC- 3'              ( 6 )
```

There is also provided in accordance with the invention a kit for carrying out the method of the invention, comprising a pair of primers for the amplification of a specific HPV DNA region according to any of Formulae 1 to 6 and a probe for the detection of the amplified DNA.

To solve the various problems mentioned above, steps taken include the selection of DNA region or regions specific to HPV 16, HPV 18, or HPV 33, within the limits of the DNA regions E6 and E7, which are ancient HPV genes present, without fail, in human cells infected with HPV, the synthesis of oligonucleotide primer DNA which is needed for the amplification of DNA of HPV by use of PCR, the isolation of cellular DNA from human cells that are infected by HPV, and amplification of these regions by PCR; it is necessary to select regions which are type-specific for the virus type and which moreover have high detection sensitivity.

We have accomplished this invention by finding that it is possible to detect HPV 16, HPV 18, and HPV 33 readily and especially to detect their presence in clinical specimens accurately by the use of a kit which contains primers and probes for the amplification and detection of a specific region and regions of the HPV.

This invention will be explained below more concretely.

HPV 16, HPV 18, and HPV 33 are previously cloned and their complete nucleotide sequences have been disclosed in Virology, 145, 181-185, 1985; Journal of Molecular Biology, 193, 599-608, 1987; and Journal of Virology, 58, 991-995, 1986.

Within the range of this E6 region, region I, which is a specific region of DNA in HPV 16, HPV 18, and HPV 33, is selected; in the same way, within the range of the E7 region, region II, which is a specific region of DNA in each of these three types of HPV, is selected (Table 1).

5

Table 1

Selected regions in E6 and E7 of HPV16, HPV18 and HPV33

Region I

5´- AAGGGCGTAACCGAAATCGGTTGAACCGAAACCGGTTAGTATAAAAGCAG

ACATTTTATGCACCAAAAGAGAACTGCAATGTTTCAGGACCCACAGGAGC        HPV16

GACCCAGAAAGTTACCACAGTTATGCACAGAGCTGCAAAC — 3´        [ 1 ]

5´- AAGGGAGTAACCGAAAACGGTCGGGACCGAAAACGGTGTATATAAAAGAT

GTGAGAAACACACCACAATACTATGGCGCGCTTTGAGGATCCAACACGGC .        HPV18

GACCCTACAAGCTACCTGATCTGTGCACGGAACTGAACAC — 3´        [ 2 ]

5´- TAGGGTGTAACCGAAAGCGGTTCAACCGAAAACGGTGCATATATAAAGCA

AACATTTTGCAGTAAGGTACTGCACGACTATGTTTCAAGACACTGAGGAA        HPV33

AAACCACGAACATTGCATGATTTGTGCCAAGCATTGGAGAC — 3´        [ 3 ]

Region II

5´- GAGGAGGATGAAATAGATGGTCCAGCTGGACAAGCAGAACCGGACAGAGC

CCATTACAATATTGTAACCTTTTGTTGCAAGTGTGACTC — 3´        HPV16        [ 4 ]

5´- GAAAACGATGAAATAGATGGAGTTAATCATCAACATTTACCAGCCCGACG

AGCCGAACCACAACGTCACACAATGTTGTGTATGTGTTGTAAGTGTGAAG        HPV18

C — 3´        [ 5 ]

5´- GAGGATGAAGGCTTGGACCGGCCAGATGGACAAGCACAACCAGCCACAGC

TGATTACTACATTGTAACCTGTTGTCACACTTGTAACAC — 3´        HPV33        [ 6 ]

For the amplification of each of these regions, it is necessary to have oligonucleotide primer DNA which is a pair of primer DNAs, one of which has about 20 residues of the sense sequence of the amplified region from the 5´ terminus and the other of which has about 20 residues of the antisense sequence from the 3´ terminal. This pair of primers should be capable of annealing with the chosen DNA region mentioned before, and as one example of these, the primer DNA shown in Table 2 can be synthesized with use of a DNA synthesizer, and purified by HPLC.

Table 2

Primer pairs for amplification of specific
regions of HPV 16, HPV 18 and HPV 33

| | | | |
|---|---|---|---|
| 5′- AAGGGCGTAACCGAAATCGGT — 3′ | p16-1 | } HPV16 Region I | [ 7 ] |
| 5′- GTTTGCAGCTCTGTGCATA — 3′ | p16-2R | | |
| 5′- AAGGGAGTAACCGAAAACGGT — 3′ | p18-1 | } HPV18 Region I | [ 8 ] |
| 5′- GTGTTCAGTTCCGTGCACA — 3′ | p18-2R | | |
| 5′- TAGGGTGTAACCGAAAGCGGT — 3′ | p33-1 | } HPV33 Region I | [ 9 ] |
| 5′- GTCTCCAATGCTTGGCACA — 3′ | p33-2R | | |
| 5′- GAGGAGGATGAAATAGATGG — 3′ | p16-3 | } HPV16 Region II | [10] |
| 5′- GAGTCACACTTGCAACAAA — 3′ | p16-4R | | |
| 5′- GAAAACGATGAAATAGATGG — 3′ | p18-3 | } HPV18 Region II | [11] |
| 5′- GCTTCACACTTACAACACA — 3′ | p18-4R | | |
| 5′- GAGGATGAAGGCTTGGACCG — 3′ | p33-3 | } HPV33 Region II | [12] |
| 5′- GTGTTACAAGTGTGACAAC — 3′ | p33-4R | | |

It is possible to purify DNA of HPV for detection from plasmids in which HPV have been cloned, from the cell lines SiHa and HeLa, which contain the DNA of HPV, and from pathological specimens of cervical carcinoma and precancerous specimens. Any of the usual methods for the purification of DNA from cells or tissues can be used. It is possible to use specimens of the following kinds: operative specimens, biopsy specimens, and smear samples from the cervix, made with the use of a cotton-tipped swab; the specimens can be prepared by paraffin fixation for the examination of pathological specimens of cervical tissue.

For the PCR method, a genetic amplification kit which includes Taq polymerase and an automated genetic amplification apparatus are commercially available from Perkin-Elmer Cetus, and with their use, it is possible to use the pair of primers of this invention for the amplification reaction of specific DNA regions.

After the amplification, it is possible to use, for example, agarose gel electrophoresis, dot hybridization and the like for the detection of DNA of HPV.

When dot hybridization is used, the different types of HPV are distinguished by the selection of probe DNA specific for a region of the DNA sequence of each type of HPV.

As the probe DNA, any probe DNA which satisfies the requirements given above can be used, and the probes listed in Table 3 give examples of such probes.

7

Table 3

Probes for the detection of HPV16, HPV18 and HPV33

Oligonucleotide probe DNA

| | | | |
|---|---|---|---|
| 5´- CATTTTATGCACCAAAAGAGAACTGCAATG — 3´ | pB16 I | HPV16 | Region I |
| 5´- TGAGAAACACACCACAATACTATGGCGCGC — 3´ | pB18 I | HPV18 | Region I |
| 5´- CATTTTGCAGTAAGGTACTGCACGACTATG — 3´ | pB33 I | HPV33 | Region I |
| 5´- CGGACAGAGCCCATTACAAT — 3´ | pB16 II | HPV16 | Region II |
| 5´- CCGAACCACAACGTCACACA — 3´ | pB18 II | HPV18 | Region II |
| 5´- CAGCCACAGCTGATTACTAC — 3´ | pB33 II | HPV33 | Region II |

The DNA of these probes can be synthesized and purified by the same methods mentioned before for primer DNA. The probe DNA can be detected with a high degree of sensitivity by labelling of the probe DNA. Any of the known methods for labelling may be employed, such as, for example, the labelling of the 5′ end of the probe DNA with $^{32}P$ with the use of T4 polynucleotide kinase, in an isotopic labelling method, and also by non-isotopic labelling methods, such as the enzymatic labelling of probe DNA, fluorescence labelling, labelling with biotin-avidin, or by the introduction of sulfone groups into the probe DNA as in Chemprobe kit (Takara), plus the use of an antibody to these groups to recognize the probe.

To establish the limit of detection of HPV DNA by this assay, the template DNA sample containing a specific amount of HPV DNA can be amplified by PCR and detected by dot hybridization. The template DNA sample can be prepared by dilution of HPV DNA from cloned plasmids with genome DNA of normal cervical tissue.

The pair of primers and the probes of this invention can be used in the detection of HPV DNA which is at the concentration of $10^{-6}$, and it is possible with their use to detect with high sensitivity regions I and II in the DNA sequence, thus making possible detection of HPV 16, HPV 18, and HPV 33 with high sensitivity.

In fact, when results from this method and those from Southern hybridization with the same sample were compared, it has been found that it is possible by our method to detect HPV DNA in the sample that could not be detected by the use of Southern hybridization. It was possible to detect HPV DNA in 84% of the samples of cervical carcinoma tissue, and it was possible to detect HPV DNA in 70% of the samples of precancerous tissue in stages CINI-CINIII, both of which rates were high, and typing of the viruses was possible.

HPV DNA was detected in samples of tissue from normal cervix at 2%, a lower rate than by the conventional methods, so that the method of this invention is particularly satisfactory in providing a standard for the detection of malignancy in cervical tissue.

The pair of primers for amplification of the specific DNA regions of this HPV and the probes for the detection of the amplified DNA regions can be supplied in kit form, and it is possible with the use of the kit to detect readily human HPV 16, HPV 18, HPV 33, or some combination of these. In addition, the pair of primers which are needed for the amplification of regions I and II may be provided in the kit in a mixture, and if the sample being tested has HPV DNA in which either region I or region II DNA has undergone mutation, it is possible to amplify the HPV DNA, and when clinical tests are being done, it is possible to prevent false-negative results. The reagents for use with the kit may be in liquid form, and they can also be in lyophilized form.

The invention will be explained in more detail by references to the following Examples.

Example 1.

Amplification and detection of HPV regions I and II by the PCR method:

(1-1) Preparation of human genome DNA:

Cells of the human cervical carcinoma cell line SiHa contain 1-10 copies of HPV 16 DNA. HeLa cells contain 10-50 copies of HPV 18 DNA. These cells were cultured separately in 6 cm culture dishes each containing Dulbecco's modified Eagle medium (DMEM; Flow Laboratories) which contains 10% fetal bovine serum (FBS; Flow Laboratories), 100 units/ml streptomycin (Meiji Seika K.K.), and 100 units/ml penicillin (Banyu Pharmaceuticals Co., Ltd.). When the cell number reached about $10^6$, the culture was stopped and the medium was removed. The cells were washed with 5 ml of TE buffer (10 mM Tris-HCl, pH 8.0, and 1mM EDTA) containing 0.1M NaCl, and then 5 ml of 0.5% SDS was added to the dishes, which were then left for 20 minutes at room temperature. The cells were scraped from the dishes and collected in polystyrene tubes. To the tubes was added 5 ml of TE buffer containing protease K at a concentration of 100 $\mu$g/ml, and the tubes were kept at 70°C for 2 hr.

Five milliliters of a mixture of equal amounts of phenol and chloroform was added to the tubes, which were then agitated gently and centrifuged at 12,000 rpm in No. 3N rotor (Tomy Seiko Co., Ltd.) for 5 min. The supernatant was obtained. To it was added 5 ml of chloroform, and the mixture was agitated gently. Centrifugation under the same conditions was done again, and the supernatant was obtained.

To this aqueous supernatant, 0.5 ml of 3 M sodium acetate and 10 ml of ethanol were added, and the mixture was mixed thoroughly. The mixture was kept at -70°C for 15 minutes, and then centrifuged for 10 minutes at 12,000 rpm in No. 3N rotor. Genome DNA was obtained as the precipitate.

The precipitate was rinsed with 80% ethanol, and dried. Then it was dissolved in 1 ml of sterilized water. About 100 $\mu$g of genome DNA was obtained.

Specimens of cervical carcinoma tissue, condyloma acuminatum tissue, and normal cervical tissue from patients who underwent hysterectomy for uterine myeloma were obtained. The specimens weighing 100 mg each were cut into pieces with dissection scissors, and the same procedures as above were followed, with treatment with protease K and then with phenol and chloroform, giving about 100 $\mu$g of genome DNA.

(1-2) Synthesis and purification of oligonucleotide primer DNA and probe DNA:

When specific DNA sequences from tissue are to be amplified by the use of the PCR method, about 20 bases of the sense sequence from the 5′ end of the region to be amplified and about 20 bases of the anti-sense sequence from the 3′ end of the region to be amplified are needed for use as the oligonucleotide primer DNA. Also, for the identification of the various types of HPV by hybridization, oligonucleotide probe DNA is needed.

The primer DNA and probe DNA shown in Tables 2 and 3, respectively, were synthesized with a DNA synthesizer (Applied Biosystems), and after deprotection, the DNA was purified by ion-exchange HPLC on TSK gel in a DEAE-2SW column. It was then desalted on a Sep-Pak C18 column (Waters), and about 50 $\mu$g of each DNA was obtained.

(1-3) Amplification of regions I and II of HPV 16 by PCR method:

From SiHa cells, HeLa cells, two specimens of cervical cancer from two patients infected with HPV 16, two specimens of condyloma acuminatum from two patients infected with HPV 6 or HPV 11, and two specimens of healthy cervical tissue from two patients who underwent hysterectomy, 1 $\mu$g of genome DNA was purified by the methods described in (1-1) and put into a 0.5 ml tube (Bio Bik). The tube was heated for 10 minutes at 94°C, and to the tube were added 10 $\mu$l of a solution provided in the Gene Amp Kit (Perkin-Elmer Cetus), which was 10x amplification buffer (100 mM Tris-HCl, pH 8.3, 500 mM KCl, 15 mM MgCl$_2$, and 0.1% (w/v) gelatin), 16 $\mu$l of a 1.25 mM dNTP mixture (dATP, dGTP, dCTP, and dTTP), 1 $\mu$l of 20 $\mu$M p16-1 primer, 1 $\mu$l of 20 $\mu$M p16-2R primer, and 0.5 $\mu$l of 5 units/$\mu$l Taq polymerase. The reaction mixture was made to 100 $\mu$l by the addition of sterilized water.

To this reaction mixture, 100 $\mu$l of mineral oil (Sigma) was added, and an automated gene amplifier (Thermal Cycler; Perkin-Elmer Cetus) was used for the amplification reaction.

The reaction was proceeded at 94°C for 1 minute for denaturation, at 55°C for 2 minutes for annealing of the primers, and at 72°C for 2 minutes for the synthetic reaction. This cycle was repeated for a total of 30 times. After the reaction, the mineral oil of the upper layer was removed. Then 10 $\mu$l of the reaction

mixture was analyzed by gel electrophoresis on a mixture of 3% Nusieve GTG agarose and 1% Sea-kem agarose (FMC). The gel was stained with ethidium bromide and the DNA bands were examined. Amplified DNA was found.

A band at the region of 140 base pairs (bp) was found with the DNA from SiHa cells and from specimens of cervical carcinoma from patients infected with HPV 16.

DNA from HeLa cells, from specimens from patients with condyloma acuminatum, and from specimens from normal cervical tissue was not amplified, so the DNA of the p16-1 and p16-2R primers specifically amplified HPV 16 DNA.

By the same methods, the primers p16-3 and p16-4R were used for the amplification of region II. Amplified DNA at the region of 89 bp was found in SiHa cells and in specimens from patients with cervical carcinoma who were infected with HPV 16.

(1-4) Identification of the HPV type by the use of dot hybridization:

The reaction mixture obtained in (1-3) was heated at 94°C for 10 minutes and then rapidly cooled in an ice bath, so that the DNA was denatured. Then 1 $\mu$l of the reaction mixture was spotted on a nylon membrane (Schleicher & Schuell) and illuminated with ultraviolet light at 254 nm for 10 minutes to fix the DNA to the membrane.

This membrane was kept in 10 ml of prehybridization buffer (5x Denhardt's solution, 5x SSC, 0.1% SDS with 100 $\mu$g/ml salmon sperm DNA) for 2 hours at 37°C for prehybridization.

Next, probe DNA pB16-1 labelled at the 5' end with $^{32}$P was added, and hybridization was carried out at 37°C for 2 hours.

The following procedure was conducted with the use of a Megalabel Kit (Takara) for labelling of the probe with $^{32}$P. First, 1 $\mu$l of probe DNA at the concentration of 10 pmol/$\mu$l was mixed with 1 $\mu$l of a x10 phosphorylation buffer, 5 $\mu$l of 10$\mu$Ci/$\mu$l [$\gamma$-$^{32}$P]ATP (Amersham), and 1 $\mu$l of T4-polynucleotide kinase (10 units), and the mixture was brought to 10 $\mu$l by the addition of 2 $\mu$l of sterilized water. The mixture was allowed to react at 37°C for 30 minutes. After the reaction, the mixture was heated at 65°C for 10 minutes, and then 2 $\mu$l of the reaction mixture (about $10^8$ cpm) was used in hybridization.

After the hybridization, the membrane was washed twice for 10 minutes each time at room temperature in washing solution 1, which contained 0.1% SDS in 2x SSC, and next washed twice for 20 minutes each time at 55°C in washing 2, which contained 0.1% SDS in 0.2x SSC. The membrane was dried and then put into a cassette containing X-ray film (Fuji Film). The cassette was kept at -70°C for 3 hours to allow the film to be exposed for autoradiography.

In this way, dots appeared which were the result of hybridization between the amplified DNA from SiHa cells and the pB16-1 probe and also between the amplified DNA from cervical carcinoma tissue infected with HPV 16 and the pB16-1 probe. No hybridization was found with the DNA from HeLa cells, tissue from condyloma acuminatum, or healthy cervical tissue.

Hybridization was done in the same way with the use of the probe pB33-I and the pB18-I. No hybridization was found. These results showed that the primers p16-I and p16-2R amplified HPV 16 specifically, and also that the probe pB16-I could detect HPV 16 by hybridization with it in a specific way.

A DNA of HPV 16 region II amplified with the use of primers p16-3 and p16-4R was hybridized specifically with probe pB16-II.

(1-5) Sensitivity of detection of HPV 16 DNA assayed with the use of regions I and II of HPV 16:

We assayed the limit of detection of HPV 16 DNA by the use of regions I and II in a model experimental system which employed cloned plasmid. The plasmid was the restriction fragment B from PstI digestion which included regions E6 and E7 of HPV 16 DNA (1776 pb; Journal of Virology, 58, 979-982, 1986) inserted into the PstI site of the vector pSV2neo (5.6 kb). The plasmid was designated p16PstIB.

The length of the entire human genome DNA is $3 \times 10^9$ bp. So that there would be 10 copies of p16PstIB/genome DNA, this plasmid was diluted in genome DNA obtained from normal cervical tissue by the methods of (1-1). In this step, 245 pg of p16PstIB was diluted with 10 $\mu$g of normal genome DNA. This mixture was diluted tenfolds with normal genome DNA, and model template DNA in which there were 10 to $10^{-6}$ copies of p16PstIB per genome DNA was prepared. Here, 1$\mu$g of genome DNA with $10^{-6}$ copies of p16PstIB corresponded to about $10^{-6}$ pg of HPV DNA, which was roughly one molecule. Then 1 $\mu$g of this model template DNA was used by the methods shown in (1-3) to amplify region I. Next, the methods of (1-4) were used for dot hybridization. Dots were found for as little as $10^{-6}$ copies/genome DNA. The same degree of sensitivity was found when region II was examined in the same way.

These results showed that with the use of regions I and II, it is possible to detect one molecule of HPV DNA ($10^{-6}$ pg) in a sample, and that the PCR method can be used for detection with extremely high sensitivity.

(1-6) Amplification and detection of regions I and II of HPV 18 and HPV 33:

Amplification and detection of KPV 18 and HPV 33 were carried out.

As template DNA, DNA (7.9 kb) containing the E6 and E7 regions of HPV 18 was inserted into the EcoRI site of the Escherichia coli plasmid pBR322, giving a plasmid designated pHPV 18(EMBO J, 3, 1151-1157, 1984). Also, as template DNA, DNA (7.9 kb) containing the E6 and E7 regions of HPV 33 was inserted into the BglII site of the E. coli plasmid plink322, giving a plasmid designated pHPV33 (J. Virol., 58, 991-995, 1986). Again, as template DNA; DNA (7.9 kb) containing the E6 and E7 regions of HPV 16 was inserted into the EcoRI site of the E. coli plasmid mentioned above, pBR322, giving plasmid pHPV16 (Proc. Natl. Acad. Sci. USA, 80, 3812-3815, 1983). Each of these template DNAs was used in an amount of 1 ng and with 1 μg of genome DNA prepared from SiHa cells or HeLa cells by the methods of (1-1).

By the methods of (1-3), a reaction system containing primer DNAs p18-1 and p18-2R or else p33-1 and p33-2R, obtained by the methods of (1-2), was used for amplification. By the methods of (1-4), dot hybridization was done; in this step, as the probe DNA, pB18-I and pB33-I obtained as described in (1-2) were used.

When amplification was done with p18-1 and p18-2R as the primers, the amplified DNA from pHPV18 or HeLa cells hybridized with the probe pB18-I, and dots appeared.

When amplification was done with p33-1 and p33-2R as the primers, only pHPV33 showed a hybridization spot with probe pB33-I. This showed that when primer DNA for the corresponding type of the virus was used, the DNA of region I was specifically amplified, and it was possible to detect it with the appropriate probe DNA.

In the same way, the different template DNAs were amplified with the use of a reaction system that contained as the primers either p18-3 and p18-4R or else p33-3 and p33-4R, which were obtained by the methods of (1-2); as the probe DNAs pB18-II and pB33-II, obtained by the methods of (1-2), were used to detect region II of HPV 18 and HPV 33. We found that with region II as well, the corresponding type of the virus was amplified, and it was possible to detect it with the appropriate probe DNA.

By the use of the methods of (1-5), the various template DNA plasmids were diluted with genome DNA obtained from healthy cervical tissue, and the appropriate pairs of primers and the appropriate probe were used for amplification and detection. In the same way as in (1-5), it was possible to detect HPV DNA at the extremely sensitive detection level of $10^{-6}$ copies/genome DNA. That is, it was possible to detect about $10^{-6}$ pg of HPV DNA in the sample, which was roughly one molecule of the HPV DNA.

The results obtained above show that the virus types HPV 16, HPV 18, and HPV 33 can be efficiently identified by the use of regions I and II of HPV, and that detection at an extremely sensitive level is possible when pairs of primers and the probe used correspond to the HPV 16, HPV 18, and HPV 33 being tested for.

Example 2.

Preparation of an amplification and detection kit for HPV 16, HPV 18, and HPV 33:

A kit was prepared for the amplification and detection of HPV 16, HPV 18, and HPV 33 in samples.

As the primer for use in the amplification of DNA; 4 μM solutions of p16-1, p16-2R, p18-1, p18-2R, p33-1, and p33-2R were dissolved separately in 100 μl of TE buffer, and these were named the HPV primer solutions I (component A). Also, p16-3, p16-4R, p18-3, p18-4R, p33-3,and p33-4R were dissolved separately in 100 μl of TE buffer, and these were named the HPV solutions II (component B). Next, p16-1, p16-2R, p16-3, p16-4R, p18-1, p18-2R, p18-3, p18-4R, p33-1, p33-2R, p33-3,and p33-4R were dissolved separately in TE buffer to the concentration of 4 μM, and these were named the HPV primer solutions III (component C).

As the probes for the detection of DNA, pB16-I, pB16-II, pB18-I, pB18-II, pB33-I, and pB33-II were dissolved separately in TE buffer, at the concentration of 2 μg in 20 μl, and these were named the HPV 16-I probe solution (component D), HPV 16-II probe solution (component E), HPV 18-I probe solution (component F), HPV 18-II probe solution (component G), HPV 33-I probe solution (component H), and HPV 33-II probe solution (component I).

Components A, D, F, and H were provided together in an amplification and detection kit I for HPV; components B, E, G, and I were provided together in an amplification and detection kit II for HPV; and

EP 0 402 132 B1

components C, D, E, F, G, H, and I were provided together in an amplification and detection kit III for HPV (Table 4).

Table 4

| Kits for the amplification and detection of HPV | | | |
|---|---|---|---|
| Kit I : | Component A | HPV primer solution I | 100 $\mu$l (5$\mu$lx20 uses) |
| | D | HPV16-I probe solution | 20 $\mu$l (1$\mu$lx20 uses) |
| | F | HPV18-I probe solution | 20 $\mu$l (1$\mu$lx20 uses) |
| | H | HPV33-I probe solution | 20 $\mu$l (1$\mu$lx20 uses) |
| Kit II : | Component B | HPV primer solution II | 100 $\mu$l (5$\mu$lx20 uses) |
| | E | HPV16-II probe solution | 20 $\mu$l (1$\mu$lx20 uses) |
| | G | HPV18-II probe solution | 20 $\mu$l (1$\mu$lx20 uses) |
| | I | HPV33-II probe solution | 20 $\mu$l (1$\mu$lx20 uses) |
| Kit III: | Component C | HPV primer solution III | 100 $\mu$l (5$\mu$lx20 uses) |
| | D | HPV16-I probe solution | 20 $\mu$l (1$\mu$lx20 uses) |
| | E | HPV16-II probe solution | 20 $\mu$l (1$\mu$lx20 uses) |
| | F | HPV18-I probe solution | 20 $\mu$l (1$\mu$lx20 uses) |
| | G | HPV18-II probe solution | 20 $\mu$l (1$\mu$lx20 uses) |
| | H | HPV33-I probe solution | 20 $\mu$l (1$\mu$lx20 uses) |
| | I | HPV33-II probe solution | 20 $\mu$l (1$\mu$lx20 uses) |

Example 3.

Detection of HPV DNA in specimens of cervical carcinoma and precancerous tissue:

By the methods of (1-1), genome DNA was obtained from about 100 mg of tissue from 43 patients who underwent surgery for cervical carcinoma and from about 10 mg of tissue obtained during biopsy of 27 patients with precancerous tissue. Of these two pooled samples, about 100 $\mu$g and about 10 $\mu$g of genome DNA were obtained, respectively.

Then, 1 $\mu$g of each of the genome DNAs was denatured by being heated at 94 °C for 10 minutes. To the denatured DNA, 10 $\mu$l of x10 buffer from the Gene Amp kit was added, as were 16 $\mu$l of 1.25 mM dNTP solution, 0.5 $\mu$l of 5 unit/$\mu$l Taq polymerase, and 5 $\mu$l of component A from the kit I of Example 2. This mixture was made to 100 $\mu$l by the addition of sterilized water. By the methods of (1-3), region I of HPV16, HPV 18, and HPV 33 were amplified together in a single tube.

After the amplification, the DNA was denatured, and 1 $\mu$l of the reaction solution was spotted on each of three nylon membranes. Three nylon membranes were prepared in the same way and hybridization was carried out by the method of (1-4).

Thus, 1 $\mu$l each of components D, F, and H described in Example 2 was used, and by the methods of (1-4), probe DNAs pB16-I, pB18-I, and pB33-I were prepared by being labelled with $^{32}$P, and the appropriate probe was added to the three membranes; followed by hybridization. Each of these types of HPV could be identified.

As shown in Table 5, of specimens of cervical carcinoma from 43 patients, HPV DNA could be detected in 36 specimens (84%).

Table 5

| Detection of HPV DNA in specimens of cervical carcinoma | | | |
|---|---|---|---|
| HPV 16 | HPV 18 | HPV 33 | Overall |
| 33/43 (77%) | 12/43 (28%) | 6/43 (14%) | 36/43 (84%) |

As shown in Table 6, specimens of precancerous cervical tissue from 27 patients, HPV DNA could be detected in 19 specimens (70%).

12

HPV DNA was detected in 5 of the 8 patients in CINI, in 7 of the 9 patients in CINII, and in 7 of the 10 patients in CINIII, so detection of HPV DNA was possible in all stages from CINI to CINIII.

The same kind of results were obtained with the use of region II. Thus, it was possible to detect regions I and II from actual pathological specimens effectively.

Table 6

| Detection of HPV DNA in specimens of precancerous cervical tissue | | | | |
|---|---|---|---|---|
| CIN | HPV16 | HPV18 | HPV33 | Overall |
| Overall | 19/27 (70%) | 6/27 (22%) | 4/27 (15%) | 19/27 (70%) |
| CINI | 5/8 | 0/8 | 2/8 | 5/8 |
| CINII | 7/9 | 2/9 | 1/9 | 7/9 |
| CINIII | 7/10 | 4/10 | 1/10 | 7/10 |

Example 4

Detection of HPV DNA in specimens of healthy cervical tissue:

About 1 mg of mucosal cells from the cervix collected with the use of cotton swabs from 42 women from areas of histologically normal cervix was treated by the methods of (1-1) to isolate genome DNA. Approximately 1 $\mu$g of DNA was obtained. By the methods of Example 3, HPV DNA was sought in the samples. In 2% of the specimens (1/42 subjects), HPV DNA was found. Thus, this method is a very effective method for the detection of malignancies in cervical tissue.

As explained in detail above, this invention provides a method for the detection with high sensitivity of HPV DNA, especially by the use of the PCR, in which regions I and II are identified, and by which method the HPV genome in cancerous tissue and precancerous tissue can be detected with high sensitivity; this invention also provides a kit which makes use of this method.

**Claims**

**1.** A method for the detection of human papilloma-virus HPV16, HPV 18, HPV 33 or any combination of these viruses by amplification of at least one HPV DNA region by use of a pair of oligonucleotide primers characterized in that the DNA region has a structure shown in Formulae 1 to 6:

Region I

5´- AAGGGCGTAACCGAAATCGGTTGAAC
CGAAACCGGTTAGTATAAAAGCAGAC
ATTTTATGCACCAAAAGAGAACTGCA
ATGTTTCAGGACCCACAGGAGCGACCC    HPV16
AGAAAGTTACCACAGTTATGCACAGA
GCTGCAAAC- 3´      ( 1 )

5´- AAGGGAGTAACCGAAACGGTCGGGA

CCGAAAACGGTGTATATAAAACATGT
GAGAAACACACCACAATACTATGGCG
CGCTTTGAGGATCCAACACGGCGACC    HPV18
CTACAAGCTACCTGATCTGTGCACGG
AACTGAACAC- 3´      ( 2 )

5´- TAGGGTGTAACCGAAAGCGGTTCAAC
CGAAACGGTGCATATATAAAGCAAA
CATTTTGCAGTAAGGTACTGCACGAC
TATGTTTCAAGACACTGAGGAAAAAC    HPV33
CACGAACATTGCATGATTTGTGCCAAG
CATTGGAGAC- 3´      ( 3 )

Region II

5´- GAGGAGGATGAAATAGATGGTCCAGCT
GGACAAGCAGAACCGGACAGAGCCCAT
TACAATATTGTAACCTTTTGTTGCAAG    HPV16
TGTGACTC- 3´      ( 4 )

5´- GAAAACGATGAAATAGATGGAGTTAAT
CATCAACATTTACCAGCCCGACGAGCC    HPV18
GAACCACAACGTCACACAATGTTGTG
TATGTGTTGTAAGTGTGAAGC- 3´ ( 5 )

5´- GAGGATGAAGGCTTGGACCGGCCAGA
TGGACAAGCACAACCAGCCACAGCTG
ATTACTACATTGTAACCTGTTGTCACA    HPV33
CTTGTAACAC- 3´      ( 6 )

14

2. A method according to claim 1 wherein the primer pairs have a structure shown in Formulae 7-12.

```
5 ´- AAGGGCGTAACC
     GAAATCGGT-3 ´  p16-1                 ⎫
                                          ⎬  HPV16, Region I
5 ´- GTTTGCAGCTCT                         ⎪          [ 7 ]
  ˝  GTGCATA-3 ´    p16-2R                ⎭


5 ´- AAGGGAGTAACC
     GAAAACGGT-3 ´  p18-1                 ⎫
                                          ⎬  HPV18, Region I
5 ´- GTGTTCAGTTCC                         ⎪          [ 8 ]
     GTGCACA-3 ´    p18-2R                ⎭


5 ´- TAGGGTGTAACC
     GAAAGCGGT-3 ´  p33-1                 ⎫
                                          ⎬  HPV33, Region I
5 ´- GTCTCCAATGCT                         ⎪          [ 9 ]
     TGGCACA-3 ´    p33-2R                ⎭
```

```
5 '- GAGGAGGATGAA
      ATAGATGG- 3 '  p16-3
                                    }  HPV16, Region II
5 '- GAGTCACACTTG                            [10]
     · CAACAAA- 3 '    p16-4R  }


5 '- GAAAACGATGAA
      ATAGATGG- 3 '  p18-3
                                    }  HPV18, Region II
5 '- GCTTCACACTTA                            [11]
     CAACACA- 3 '    p18-4R  }


5 '- GAGGATGAAGGC
      TTGGACCG- 3 '  p33-3
                                    }  HPV33, Region II
5 '- GTGTTACAAGTG                            [12]
     TGACAAC- 3 '    p33-4R  }
```

3. A kit for carrying out the method of human papilloma-virus detection of claim 1 comprising a pair of primers for the amplification of a specific HPV DNA region according to any of Formulae 1 to 6 of claim 1 and a probe for the detection of the amplified DNA.

**Patentansprüche**

1. Verfahren zum Nachweis von humanem Papillomvirus HPV 16, HPV 18, HPV 33 oder jeder Kombination dieser Viren durch Amplifizierung von zumindest einer HPV-DNA-Region mittels eines Oligonukleotid-Primerpaares, dadurch gekennzeichnet, daß die DNA-Region eine in den Formeln 1 bis 6 gezeigte Struktur besitzt:

Region I

5´- AAGGGCGTAACCGAAATCGGTTGAAC
CGAAACCGGTTAGTATAAAAGCAGAC
ATTTTATGCACCAAAAGAGAACTGCA
ATGTTTCAGGACCCACAGGAGCGACCC     HPV16
AGAAAGTTACCACAGTTATGCACAGA
GCTGCAAAC-3´       ( 1 )

5´- AAGGGAGTAACCGAAAACGGTCGGGA

CCGAAAACGGTGTATATAAAAGATGT
GAGAAACACACCACAATACTATGGCG
CGCTTTGAGGATCCAACACGGCGACC     HPV18
CTACAAGCTACCTGATCTGTGCACGG
AACTGAACAC-3´      ( 2 )

5´- TAGGGTGTAACCGAAAGCGGTTCAAC
CGAAAACGGTGCATATATAAAGCAAA
CATTTTGCAGTAAGGTACTGCACGAC
TATGTTTCAAGACACTGAGGAAAAAC     HPV33
CACGAACATTGCATGATTTGTGCCAAG
CATTGGAGAC-3´      ( 3 )

Region II

5´- GAGGAGGATGAAATAGATGGTCCAGCT
GGACAAGCAGAACCGGACAGAGCCCAT
TACAATATTGTAACCTTTTGTTGCAAG     HPV16
TGTGACTC-3´      ( 4 )

5´- GAAAACGATGAAATAGATGGAGTTAAT

17

```
         1 3 0              1 4 0
CATCAACATTTACCAGCCCGACGAGCC   HPV18
                1 6 0           1 7 0
GAACCACAACGTCACACAATGTTGTG
1 8 0           1 9 0
TATGTGTTGTAAGTGTGAAGC-3'  [ 5 ]
```

```
              1 0 0              1 1 0
5'-GAGGATGAAGGCTTGGACCGGCCAGA
1 2 0              1 3 0
TGGACAAGCACAACCAGCCACAGCTG
     1 4 0           1 5 0           1 6 0
ATTACTACATTGTAACCTGTTGTCACA   HPV33
          1 7 0
CTTGTAACAC-3'              [ 6 ]
```

**2.** Verfahren nach Anspruch 1, worin die Primerpaare eine Struktur besitzen, die in den Formeln 7-12 gezeigt ist:

```
5'- AAGGGCGTAACC
       GAAATCGGT-3'  p16-1

                              HPV16, Region I
5'- GTTTGCAGCTCT                   [ 7 ]
       GTGCATA-3'    p16-2R
```

```
5'- AAGGGAGTAACC
       GAAAACGGT-3'  p18-1.

                              HPV18, Region I
5'- GTGTTCAGTTCC                   [ 8 ]
       GTGCACA-3'    p18-2R
```

```
5'- TAGGGTGTAACC
       GAAAGCGGT-3'  p33-1

                              HPV33, Region I
5'- GTCTCCAATGCT                   [ 9 ]
       TGGCACA-3'    p33-2R
```

```
5'- GAGGAGGATGAA
       ATAGATGG-3'  p16-3

                              HPV16, Region II
5'- GAGTCACACTTG                   [ 10 ]
       CAACAAA-3'    p16-4R
```

18

```
5 ´- GAAAACGATGAA
     ATAGATGG- 3 ´  p18-3          ⎫
                                   ⎬  HPV18, Region II
5 ´- GCTTCACACTTA                  ⎪         ( 11 )
     CAACACA- 3 ´   p18-4R         ⎭


5 ´- GAGGATGAAGGC
     TTGGACCG- 3 ´  p33-3          ⎫
                                   ⎬  HPV33, Region II
5 ´- GTGTTACAAGTG                  ⎪         ( 12 )
     TGACAAC- 3 ´   p33-4R         ⎭
```

3. Kit zum Ausführen des Verfahrens zum Nachweis des humanen Papillomvirus nach Anspruch 1, wobei der Kit ein Primerpaar zur Amplifizierung einer spezifischen HPV-DNA-Region gemäß einer der Formeln 1 bis 6 von Anspruch 1 und eine Sonde für den Nachweis der amplifizierten DNA umfaßt.

**Revendications**

1. Procédé de détection des virus de papillome humain HPV16, HPV 18, HPV 33 ou de n'importe quelle association de ces virus par amplification d'au moins une région d'ADN de HPV en utilisant une paire d'amorces oligonucléotidiques, caractérisé en ce que la région d'ADN possède une structure représentée dans les formules 1 à 6 :

Region I

5'- AAGGGCGTAACCGAAATCGGTTGAAC

CGAAACCGGTTAGTATAAAAGCAGAC

ATTTTATGCACCAAAAGAGAACTGCA

ATGTTTCAGGACCCACAGGAGCGACCC          HPV16

AGAAAGTTACCACAGTTATGCACAGA

GCTGCAAAC-3'                    ( 1 )

5'- AAGGGAGTAACCGAAAACGGTCGGGA


CCGAAAACGGTGTATATAAAAGATGT

GAGAAACACACCACAATACTATGGCG

CGCTTTGAGGATCCAACACGGCGACC          HPV18

CTACAAGCTACCTGATCTGTGCACGG

AACTGAACAC-3'                  ( 2 )

5'- TAGGGTGTAACCGAAAGCGGTTCAAC

CGAAAACGGTGCATATATAAAGCAAA

CATTTTGCAGTAAGGTACTGCACGAC

TATGTTTCAAGACACTGAGGAAAAAC          HPV33

CACGAACATTGCATGATTTGTGCCAAG

CATTGGAGAC-3'                  ( 3 )

Région II

5'- GAGGAGGATGAAATAGATGGTCCAGCT

GGACAAGCAGAACCGGACAGAGCCCAT

TACAATATTGTAACCTTTTGTTGCAAG          HPV16

TGTGACTC-3'                    ( 4 )


5'- GAAAACGATGAAATAGATGGAGTTAAT

CATCAACATTTACCAGCCCGACGAGCC          HPV18

GAACCACAACGTCACACAATGTTGTG

TATGTGTTGTAAGTGTGAAGC-3'     ( 5 )

5'- GAGGATGAAGGCTTGGACCGGCCAGA

TGGACAAGCACAACCAGCCACAGCTG          HPV33

ATTACTACATTGTAACCTGTTGTCACA

CTTGTAACAC-3'                  ( 6 )

**2.** Procédé suivant la revendication 1, dans lequel les paires d'amorces possèdent une structure représentée dans les formules 7 à 12.

```
5 '- AAGGGCGTAACC
      GAAATCGGT-3 '  p16-1
                             }  HPV16, Région I
5 '- GTTTGCAGCTCT
   -  GTGCATA-3 '    p16-2R         [ 7 ]


5 '- AAGGGAGTAACC
      GAAAACGGT-3 '  p18-1
                             }  HPV18, Région I
5 '- GTGTTCAGTTCC
      GTGCACA-3 '    p18-2R         [ 8 ]


5 '- TAGGGTGTAACC
      GAAAGCGGT-3 '  p33-1
                             }  HPV33, Région I
5 '- GTCTCCAATGCT
      TGGCACA-3 '    p33-2R         [ 9 ]
```

```
5'- GAGGAGGATGAA
     ATAGATGG-3'  p16-3
                              ⎞
                              ⎟   HPV16, Région II
5'- GAGTCACACTTG              ⎟        (10)
     · CAACAAA-3'   p16-4R    ⎠


5'- GAAAACGATGAA
     ATAGATGG-3'  p18-3
                              ⎞
                              ⎟   HPV18, Région II
5'- GCTTCACACTTA              ⎟        (11)
     CAACACA-3'   p18-4R      ⎠


5'- GAGGATGAAGGC
     TTGGACCG-3'  p33-3
                              ⎞
                              ⎟   HPV33, Région II
5'- GTGTTACAAGTG              ⎟        (12)
     TGACAAC-3'   p33-4R      ⎠
```

3. Kit pour la mise en oeuvre du procédé de détection de virus de papillome humain suivant la revendication 1, comprenant une paire d'amorces pour l'amplification d'une région d'ADN de HPV spécifique répondant à l'une quelconque des formules 1 à 6 suivant la revendication 1 et une sonde pour la détection de l'ADN amplifié.